# EUROPÄISCHE PATENTSCHRIFT

(11) **EP 1 352 059 B1**
(45) Veröffentlichungstag und Bekanntmachung des Hinweises auf die Patenterteilung: **09.11.2005**
(21) Anmeldenummer: 01273288.9
(22) Anmeldetag: 24.12.2001
(51) Int. Cl.: C12N 15/10, C12N 1/06

(54) **VERFAHREN ZUR AUFBEREITUNG VON BIOMASSE ZUR HERSTELLUNG VON PLASMID DNA HALTIGEM ZELLLYSAT**
METHOD FOR TREATING BIOMASS FOR PRODUCING CELL LYSATE CONTAINING PLASMID DNA
PROCEDE DE TRAITEMENT DE BIOMASSE POUR LA PRODUCTION DE LYSAT CELLULAIRE CONTENANT DE L'ADN PLASMIDIQUE

(30) Priorität: 19.01.2001 CH 80012001
(43) Veröffentlichungstag der Anmeldung: 15.10.2003
(73) Patentinhaber: DRM, DR. MÜLLER AG, CH-8708 Männedorf ZH (CH); Polytag Technology SA, 1015 Lausanne (CH)
(72) Erfinder: SCHUMACHER, Ivo, CH-8645 Jona (CH); FREITAG, Ruth, CH-1015 St-Sulpice (CH); HILBRIG, Frank, CH-1012 Lausanne (CH)
(74) Vertreter: Herrmann, Peter Johannes
(86) Internationale Anmeldenummer: PCT/CH2001/000741
(87) Internationale Veröffentlichungsnummer: WO 2002/057446

(56) Entgegenhaltungen:
- EP-A- 0 648 776
- EP-A- 0 696 638
- EP-A- 0 795 602
- EP-A- 0 814 156
- WO-A-92/07863
- WO-A-95/14768
- WO-A-96/36706
- US-A- 5 234 809
- WANG B ET AL.: "Large-scale preparation of plasmid DNA by microwave lysis" BIOTECHNIQUES, Bd. 18, Nr. 4, April 1995 (1995-04), Seiten 554-555, XP002169027

## Beschreibung

Die vorliegende Erfindung betrifft ein Verfahren zur integrierten Aufbereitung von Biomasse aus einem Zellkulturprozess zur Herstellung von klarem, Plasmid DNA haltigem Zelllysat, sowie ein Plasmid DNA Zelllysat, hergestellt nach dem Verfahren.

Unter Aufbereitung ist ein Konditionierungsverfahren zur Bereitstellung von klarem, Plasmid DNA haltigem Zelllysat zu verstehen. Unter einem integrierten Verfahren soll ein Verfahren verstanden werden, in welchem die einzelnen Verfahrensschritte ineinander übergehen, so dass der Produktstrom praktisch kontinuierlich geführt wird. Das an sich mehrstufige Verfahren kann kontinuierlich oder chargenweise durchgeführt werden.

Die Abtrennung von biologischem Material aus einem Zellkulturprozess, der Aufschluss des biologischen Materials und die Herstellung eines klaren, Plasmid DNA haltigen Zelllysats ist im Bereich der Molekularbiologie eine gebräuchliche Methode. Bei den bekannten Verfahren wird biologisches Material, beispielsweise aus E.coli Bakterienzellen, vom Kulturüberstand abgetrennt und nach Wiederaufschlämmung aufgeschlossen. Nach Abtrennung der festen Bestandteile wird ein sogenanntes klares Zelllysat bereitgestellt, das neben genomischer DNA, RNA, Proteinen und Endotoxinen die Plasmid DNA enthält.

Es ist bekannt, biologisches Material durch chargenweises oder kontinuierliches Zentrifugieren aus dem Zellkulturprozess abzutrennen. Die Abtrennung von Backhefezellen durch Filtration über ein Bett von Filterhilfsmittel wird in GB-A-1082862 beschrieben. Danach ist auch die Abtrennung von Zellrückständen mittels Filterhilfsmittel aus einem Hefeautolysat bekannt.

Die üblichen Methoden zum Zellaufschluss sind die bekannte alkalische Lyse und die thermische Lyse. Um den Zellaufschluss zu verbessern werden häufig Enzyme, beispielsweise Lysozym, und/oder Detergenzien zur Zellsuspension zugegeben. Bei dem alkalischen Lyseverfahren wird nach dem Zellaufschluss bei pH 12 durch Zugabe von Natronlauge und Natriumdodecylsulfat und anschliessendem Neutralisieren mit einem hochmolaren Acetatpuffer ein Präzipitat erhalten, das im wesentlichen die Zelldebris und Teile der genomischen DNA und der Proteine enthält. Die vollständige Abtrennung dieses Präzipitats ist nur durch intensives Zentrifugieren zu erreichen. Eine Zentrifugalbeschleunigung von 12'000 g reicht dazu nicht aus (I. Feliciello et al., Anal. Biochem. 212 (1993) 394-401). Erst bei einer Zentrifugalbeschleunigung von 26'000 g für 30 Minuten bei 4°C kann das Präzipitat weitestgehend abgetrennt werden. Die Filtration dieses Präzipitats ist dagegen auch unter Zuhilfenahme feinster Filterhilfsmittel oder in Kombination mit einem Flottierungsschritt mit niedrigen Prozessraten und hohen Verlusten von Plasmid DNA verbunden. Die Adsorption von Plasmid DNA an der Filterhilfsmitteloberfläche trägt beträchtlich zu den Verlusten bei. Insbesondere Plasmid DNA bindet sehr schnell und fest an solchen Mineraloberflächen, wenn die Konzentration zweiwertiger Kationen 0.1 mmol oder die einwertiger Kationen 20 mmol im Falle von Kalium oder 50 mmol im Falle von Natrium übersteigt (G. Romanowski et al., Appl. Environ. Microbiol. 57 (1991) 1057-1061).

Hochmolekulare Nukleinsäuren sind empfindlich gegenüber Scherkräften. Starke Scherkräfte können zu irreversiblen Schädigungen von Nukleinsäuren, insbesondere zu Strangbrüchen führen. Aus diesem Grund werden mechanische Aufschlussverfahren für die Nukleinsäure-Aufbereitung selten eingesetzt (A. Carlson et al., Biotechnol. Bioeng. 48 (1995) 303-315). Es ist auch bekannt, dass bei industriellen Zentrifugen mit kontinuierlichem Flüssigkeitseintrag hohe Scherkräfte am Rotoreingang auf die Nukleinsäuren wirken, die unweigerlich zu Strangbrüchen führen.

In der WO 96/36706 wird ein Verfahren beschrieben, bei dem Mikroorganismen in Gegenwart von Detergenzien (Triton®) und durch Erhitzen auf 70°C bis 100°C in einem Durchflusswärmetauscher aufgeschlossen werden. Es handelt sich um ein Kombinationsverfahren aus Thermolyse und mit einem Detergenz (optional plus Enzym). Ohne Lysozym wird eine Temperaturbehandlung während 30 sec beschrieben; mit Lysozym eine solche von 6 sec. Beim Zellaufschluss durch Erhitzen bilden sich feste Verbindungen aus Debris, genomischer DNA und Proteinen. Zudem darf durch die Hitzebehandlung eine weittestgehende Denaturierung von DNA abbauenden Enzymen, sogenannten DNasen, angenommen werden. Ein klares Zelllysat wird nach chargenweisem Zentrifugieren erhalten. In diesem Verfahren wird nur der Zellaufschluss kontinuierlich durchgeführt, die Abtrennung der festen Bestandteile erfolgt chargenweise durch Zentrifugieren. Um das endgültige klare Zelllysat zu erhalten, musste im Anschluss an die Zentrifugation noch eine Filtration über einen Membranfilter durchgeführt werden.

Nach WO 92 07863 sind eine Vorrichtung und ein Verfahren zu Isolierung von Nukleinsäuren aus Zellsuspensionen bekannt. Danach werden die Zellen in den Hohlräumen einer vorgelegten, porösen Matrix in Form einer Schicht immobilisiert. Dies wird durch Tiefenfiltration in der Matrix erreicht, indem die Hohlraumgrösse in der Grössenordnung der Zellen liegt und die Matrixoberfläche Ionenaustauschereigenschaften aufweist. Die Partikelgrösse der Matrix beträgt 10 bis 50 µm. Aufgrund der Ionenaustauschereigenschaften absorbiert DNA an der Matrixoberfläche. Eine Absorption der DNA ist nicht Gegenstand der Erfindung. Das bekannte Verfahren liefert ein aufgereinigtes DNA, nicht jedoch ein klares, Plasmid DNA.

Nach der EP-A-0814156 wird ein Verfahren zur Reinigung von DNA beschrieben. Die darin verwendeten Kieselguren sind nicht näher spezifiziert. Die Lyse erfolgt nach dem alkalischen Verfahren. Eine thermische Lyse wird nicht beschrieben. Lyse erfolgt nach dem alkalischen Verfahren. Eine thermische Lyse wird nicht beschrieben.

Aus dem Stand der Technik sind somit weder Verfahren bekannt, die es ermöglichen, grosse Mengen Biomasse integriert aufzuarbeiten, noch sind Verfahren bekannt, mit denen integriert grosse Mengen Plasmid DNA haltige, klare Zelllysate bereitgestellt werden können.

Es besteht ein Bedarf nach einem effizienten Konditionierungsverfahren zur Bereitstellung von klarem, Plasmid DNA haltigem Zelllysat aus einem Zellkulturprozess, das die Plasmid DNA Aufreinigung ermöglicht.

Die der vorliegenden Erfindung zugrundeliegende Aufgabe ist es daher, ein integriertes Aufarbeitungsverfahren bereitzustellen, das es auf effiziente und kontrollierte Weise unter Vermeidung der Nachteile des alkalischen Lyseverfahrens und der Zentrifugation ermöglicht, grosse Mengen klarer Zelllysate mit hoher Plasmid DNA Ausbeute aus einer Biomasse bereitzustellen.

Diese Aufgabe wird erfindungsgemäss dadurch gelöst, dass jeweils in Gegenwart eines Filterhilfsmittels in einer ersten Stufe in einem Filter filtriert, in einer zweiten Stufe die im Filterkuchen enthaltene Biomasse thermisch aufgeschlossen und in einer weiteren Stufe das Zelllysat filtriert wird.

Es wurde nun festgestellt, dass nach dem Verfahren der vorliegenden Erfindung schnell jede Volumenmenge, von 50 ml bis 10'000 l, Biomasse zu klarem Zelllysat, das die Plasmid DNA enthält, verarbeitet werden kann. Besonders vorteilhaft ist, dass mit dem Verfahren der vorliegenden Erfindung über den angelegten Überdruck beim Filtrieren eine Kontrolle über die auf die Nukleinsäuren wirkenden Scherkräfte gegeben ist. Insbesondere zeigt ein erfindungsgemäss hergestelltes Zelllysat nach gelelektrophoretischer Auftrennung neben den Banden für genomische DNA und RNA deutlich die Banden für Plasmid DNA, ohne erkennbare Schädigung der Nukleinsäuren infolge Strangbrüchen. Ausserdem ist das erhaltene Zelllysat durch die Hitzebehandlung von unangenehmen Gerüchen befreit.

Es ist zweckmässig, dass das Filterhilfsmittel während des Aufschlusses vorhanden ist. Das hat den Vorteil, dass das für die folgende Klarfiltration des Zelllysats benötigte Filterhilfsmittel bereits vorhanden ist. Die kontinuierliche Nutzung desselben Filterhilfsmittels über alle Teilschritte (Abtrennung, Aufschluss, Klärung) ist zudem verfahrenstechnisch effizient und damit wirtschaftlich. Besonders vorteilhaft ist, wenn anstatt den Filterkuchen für die Lyse aufzuschlämmen, unmittelbar im existierenden Filterkuchen kombiniert thermisch aufgeschlossen und klarfiltriert wird (zum Beispiel durch Durchpumpen von heissem Lysepuffer) und somit direkt sehr klares Zelllysat gewonnen werden kann. Damit wird das Gesamtverfahren durch die Reduktion der Zahl der Einzelverfahrensschritte noch effizienter und damit ökonomisch noch günstiger.

Die Auswahl des Filterhilfsmittels ist von entscheidender Bedeutung. Das Filterhilfsmittel muss inert sein, um Adsorptionseffekte zu minimieren. Es hat sich als zweckmässig erwiesen, als Filterhilfsmittel kalzinierte Kieselguren mit einem SiO₂-Gehalt von wenigstens 90 Gew.-% und zusätzlich folgenden Eigenschaften zu verwenden:
- Nassdichte: 0.2 - 0.4 g/cm³
- Permeabilität: 0.02 - 2 Darcy
- BET Oberfläche: 2 - 20 m²/g
- Geometrische Oberfläche: 0.25 - 0.65 m²/g
- Partikelgrösse 2.5 - 8.0 µm
- Partikelgrössenretention (99%): 0.1 - 2 µm

Das hat den Vorteil, dass in einem Filtrationsschritt sehr klares Filtrat mit ausreichend hohem Filtrationsfluss bei moderatem Differentialdruck erhalten wird. Neben dieser Kombination aus Effizienz und Effektivität bietet die Verwendung hochreiner Filterhilfsmittel den Vorteil, dass deren Oberflächen standardisiert sind, und damit deren Eigenschaften kontrolliert werden können.

Es ist gleichfalls zweckmässig, dass in allen Stufen dasselbe Filterhilfsmittel verwendet wird. Das hat den Vorteil, dass die einzelnen Verfahrensschritte ineinander übergehen, so dass der Produktstrom praktisch kontinuierlich geführt wird.

Die Menge an Filterhilfsmittel, bezogen auf den Feststoffgehalt der zu filtrierenden Lösung, die Filtrationsfläche und die Höhe des maximal anzulegenden Filtrationsüberdrucks kann nur empirisch bestimmt werden.

Es hat sich als besonders zweckmässig erwiesen, den Aufschluss thermisch durchzuführen. Das hat den Vorteil, dass der Aufschluss schonend, beispielsweise im physiologischen pH-Bereich, unter sehr gut steuerbaren und reproduzierbaren Bedingungen durchgeführt werden kann. Die Prozessrate kann erheblich gesteigert werden, wenn anstelle der chargenweisen Erhitzung der Zellsuspension ein Durchflusswärmetauscher verwendet wird. Es ist ausserdem anzunehmen, dass beim Erhitzen ein Teil der DNA-abbauenden Enzyme inaktiviert wird. Im Vergleich zur alkalischen Lyse ist die Geruchsbelästigung bei der thermischen Lyse deutlich geringer.

Der Aufschluss wird bei Temperaturen zwischen 70°C und 90°C, bevorzugt bei 70°C bis 85°C, insbesondere bei 80°C durchgeführt. Bei einer Temperatur von weniger als 70°C ist der thermische Aufschluss unvollständig; bei einer Temperatur über 90°C schmilzt Plasmid DNA. Die Dauer der thermischen Behandlung kann nur empirisch bestimmt werden. Sie beträgt 30 Sekunden bis einige Minuten.

Der Aufschluss muss bei einem pH-Wert zwischen 7 und 10 durchgeführt werden. In diesem pH-Bereich sind die Adsorptionsverluste an Plasmid DNA minimal und die Aktivität von Lysozym optimal. Ausserdem liegt Plasmid DNA im gewonnenen klaren Zelllysat bereits im physiologischen pH-Bereich vor.

Es ist zweckmässig, den Aufschluss in Gegenwart von ausschliesslich einwertigen Kationen durchzuführen. Das hat den Vorteil, dass die Verluste bei der abschliessenden Filtration zum klaren Plasmid DNA-Zelllysat infolge von Adsorption an der Filterhilfsmitteloberfläche minimal sind. Mehrwertige Kationen, die während des Zellaufschlusses freigesetzt werden, werden durch Komplexierungsmittel maskiert.

Die maximale Konzentration an Kationen soll höchstens 20 mmol für K⁺, höchstens 50 mmol für Na⁺ oder höchstens 150 mmol für NH₄⁺ betragen. Bei Überschreiten dieser Maximalkonzentrationen treten Verluste von Plasmid DNA infolge von Adsorption an der Filterhilfsmitteloberfläche auf. Sind mehrere Kationenspezies vorhanden, ist bei der Festlegung der zulässigen Gesamtkonzentration die Additivität der Effekte zu berücksichtigen.

Klares, Plasmid DNA haltiges Zelllysat zeichnet sich durch eine Klarheit OD₆₀₀ von höchstens 0.05 E/cm, entsprechend einer Abnahme der Trübung von mindestens 99 %, aus. Das erhaltene klare Zelllysat mit der Plasmid DNA kann zum Klonieren, zur Transformation, zur Transfektion, zur Mikroinjektion in Zellen, zur Gentherapie, zur DNA Vakzinierung und /oder zur Polymerasen-Ketten-Reaktion (PCR) verwendet werden.

Die Erfindung soll anhand von Ausführungsbeispielen näher beschrieben werden.

### Beispiel 1

Plasmid DNA pHEN1 (4618 bp) im E.coli Stamm TG1 wurde im 2TY Medium bei 37°C für 16 Stunden im Schüttelkolben vermehrt. Zu drei E.coli Suspensionen von jeweils 125 ml (OD₆₀₀ = 6) wurde CelpureP300® (Markenzeichen der Firma World Minerals Inc.) mit 15, 30 bzw. 60 g/l zugegeben und kontinuierlich bei 20°C gerührt. Anschliessend wurde jede Suspension in einen Nutschefilter (10 cm² Filterfläche, 200 ml Füllvolumen) gegeben, in dem zuvor ein Vorfilterkuchen von 2 kg/m² CelpureP300® über einem Filtermedium aus Polypropylen Monofilament (Porengröße 10 µm) aufgebaut worden war. Die Filtration wurde bei 0.5 bar Überdruck und 20°C durchgeführt.

Es wurde in allen Fällen ein klares Filtrat erhalten. Der höchste mittlere Filtrationsfluss (2.8 m³/m²h) wurde mit 30 g/l CelpureP300® erreicht.
Die Ergebnisse sind in Tabelle 1 zusammengefasst.

**Tabelle 1**

| CelpureP300® (*)in g/l | Mittlerer Filtrationsfluss (m³/m²h) | OD₆₀₀ des Filtrats (E/cm) | Abnahme der Trübung in % |
|---|---|---|---|
| 60 | 1.8 | 0.04 | 99 |
| 30 | 2.8 | 0.05 | 99 |
| 15 | 2.4 | 0.03 | 99 |

| | | | |
|---|---|---|---|
| (*) Eigenschaften: Siliziumdioxidanteil: 98.65%; Nassdichte: 0.26 g/cm³. Permeabilität: 0.15 - 0.30 Darcy: BET Oberfläche: 4 - 6 m²/g; Geometrische Oberfläche: 0.62 m²/g; Partikelgrössenretention (99%): 0.6 - 0.75 µm. | | | |

### Beispiel 2:

Lysepuffer A:
   150 mmol Tris HCl, 25 mmol Na₂EDTA, 8 Gew.-% Saccharose (pH 9)
Lysepuffer B:
   wie Lysepuffer A, zusätzlich 2% TritonX-100® (Markenzeichen der Firma Röhm und Haas)

Zwei Filterkuchen aus dem Beispiel 1 mit 30g/l CelpureP300® wurden jeweils in 190 ml Lysepuffer A bzw. B resuspendiert. 500 U/ml Lysozym wurde jeweils zugegeben, und die Suspensionen wurden auf 80°C für 30 Sekunden unter kontinuierlichem Rühren aufgeheizt. Die noch heissen Suspensionen wurden in einen Nutschefilter gegeben, in dem zuvor ein Vorfilterkuchen von 2 kg/m² CelpureP300® (inkubiert für 15 Minuten im entsprechenden Lysepuffer) über einem Filtermedium aus Polypropylen Monofilament (Porengröße 10 µm) aufgebaut worden war. Die Filtration wurde bei 0.5 bar Überdruck durchgeführt.

Mittels des Lysepuffers A wurde ein klares Zelllysat (OD₆₀₀ = 0.02) mit einem mittleren Filtrationsfluss von 0.4 m³/m²h erhalten. Durch die Zugabe von Triton X-100® (Lysepuffer B) war demgegenüber der mittlere Filtrationsfluss um 50% reduziert. Eine signifikante Erhöhung des mittleren Filtrationsflusses auf 1.5 m³/m²h im Lysepuffer A wurde erreicht, als die Gesamtmenge CelpureP300® auf 100 g/l eingestellt worden war und als PVDF Kette/PTFE Schuss Monofilament (Porengröße 11.5 µm) als Filtermedium benutzt worden war.

### Beispiel 3

3.7ml λ DNA (E.coli; 48.5 kb dsDNA; 0.51 mg/ml) wurden in 25 ml einer Pufferlösung bestehend aus 250 mmol Tris·HCl, 25 mmol Na₂EDTA, 8 Gew.-% Saccharose (pH 9.09) gegeben. 0.75 g CelpureP300® (30 g/l) wurden zugegeben, und die Suspension wurde für 30 Minuten bei 20°C und 200 rpm geschüttelt.

UV Absorptionsmessungen bei 260 nm und ein PicoGreen® Test (Kit der Firma Molekular Probes Inc.) ergaben keinen Unterschied zwischen der λ DNA Konzentration im Überstand der Suspension und der Konzentration in der Ausgangslösung.

### Beispiel 4

E.coli Zellen DH5α mit der Plasmid DNA pEGFP-N1 wurden zu vier gleichen Teilen in einem Puffer bestehend aus TrisHCl und 10 mM EDTA suspendiert. Aus einer dieser Suspensionen wurden die Zellen mit dem Nucleobond® AX Kit (Markenzeichen der Firma Macherey-Nagel) nach dem alkalischen Lyseverfahren aufgeschlossen, und die Plasmid DNA isoliert und aufgereinigt. Bei den anderen .. drei Suspensionen wurden die Zellen thermisch für 5 Minuten bei 70°C/ pH 9, 70°C/ pH 10 bzw. 60°C/ pH 10 aufgeschlossen, und die Plasmid DNA wurde jeweils mit dem gleichen Kit isoliert und aufgereinigt. Die Plasmid DNA Quantifizierung der vier Proben mittels des PicoGreen Tests ergab, dass der Aufschluss nach dem alkalischen Lyseverfahren und die thermische Lyse bei 70°C und pH 9 bzw. pH 10 zu gleichen Ausbeuten an Plasmid DNA führte, während der thermische Aufschluss bei 60°C und pH 10 nur etwa 30% der Ausbeute der nach den drei anderen Verfahren erhaltenen Plasmid DNA lieferte. Aus Beispiel 3 ist bekannt, dass unter den beschriebenen thermischen Lysebedingungen keine Adsorption von Plasmid DNA an der Filterhilfsmitteloberfläche zu erwarten ist.

### Messverfahren:

### Klarheitsmessung

Die Klarheit eines Filtrats (optische Dichte, OD) wurde mit einem UV/Vis Spektrometer Lambda 20 der Firma Perkin-Elmer im Absorptionsmodus bei 600 nm und einer Weglänge von 1 cm gegen Wasser als Referenz bei Raumtemperatur bestimmt.

## Patentansprüche

1. Verfahren zur integrierten Aufbereitung von Biomasse aus einem Zellkulturprozess zur Herstellung von klarem, Plasmid DNA haltigem Zelllysat, **dadurch gekennzeichnet, dass** jeweils in Gegenwart eines inerten Filterhilfsmittels in einer ersten Stufe in einem Filter filtriert, in einer zweiten Stufe die im Filterkuchen enthaltene Biomasse thermisch aufgeschlossen und in einer weiteren Stufe das Zelllysat filtriert wird.

2. Verfahren nach Anspruch 1, **dadurch gekennzeichnet, dass** der Aufschluss bei 70°C bis 90°C durchgeführt wird.

3. Verfahren nach Anspruch 1, **dadurch gekennzeichnet, dass** der thermische Aufschluss während wenigstens 30 Sekunden durchgeführt wird.

4. Verfahren nach Anspruch 1, **dadurch gekennzeichnet, dass** als inertes Filterhilfsmittel Kieselguren mit folgenden Eigenschaften verwendet werden:
• Siliziumdioxidanteil: ≥ 90 %
• Nassdichte: 0.2 - 0.4 g/cm³
• Permeabilität: 0.02 - 2 Darcy
• BET Oberfläche: 2 - 20 m²/g
• Geometrische Oberfläche: 0.25 - 0.65 m²/g
• Partikelgrösse 2.5 - 8.0 µm
• Partikelgrössenretention (99%): 0.1 - 2 µm

5. Verfahren nach den Ansprüchen 1 bis 4, **dadurch gekennzeichnet, dass** in allen drei Stufen dasselbe Filterhilfsmittel verwendet wird.

6. Verfahren nach den Ansprüchen 1 bis 5, **dadurch gekennzeichnet, dass** der Aufschluss bei einem pH-Wert zwischen 7 und 10 durchgeführt wird.

7. Verfahren nach den Ansprüchen 1 bis 6, **dadurch gekennzeichnet, dass** der Aufschluss in Gegenwart von ausschliesslich einwertigen Kationen durchgeführt wird.

8. Verfahren nach den Ansprüchen 1 bis 7, **dadurch gekennzeichnet, dass** die maximale Konzentration an Kationen höchstens 20 mmol für K⁺, höchstens 50 mmol für Na⁺ oder höchstens 150 mmol für NH₄⁺ ist

## Claims

1. Method for the integrated treatment of biomass from a cell culture process for producing clear cell lysate containing plasmid DNA, **characterized in that** in each case filtration is effected in a first stage in a filter in the presence of a filtering agent, the biomass contained in the filter cake is thermally digested in a second stage and the cell lysate is filtered in a further stage.

2. Method according to Claim 1, **characterized in that** the digestion is carried out at 70°C to 90°C.

3. Method according to Claim 1, **characterized in that** the thermal digestion is carried out for at least 30 seconds.

4. Method according to Claim 1, **characterized in that** the filtering agents used are kieselguhrs having the following properties:
• Silica content: ≥ 90%
• Wet density: 0.2 - 0.4 g/cm³
• Permeability: 0.02 Darcy
• BET surface area: 2 - 20 m²/g
• Geometrical surface area: 0.25 - 0.65 m²/g
• Particle size: 2.5 - 8.0 µm
• Particle size retention (99%): 0.1 - 2 µm

5. Method according to any of Claims 1 to 4, **characterized in that** the same filtering agents is used in all three stages.

6. Method according to any of Claims 1 to 5, **characterized in that** the digestion is carried out at a pH-1 between 7 and 10.

7. Method according to any of Claims 1 to 6, **characterized in that** the digestion is carried out in the presence of exclusively monovalent cations.

8. Method according to any of Claims 1 to 7, **characterized in that** the maximum concentration of cations is not more than 20 mmol for K+, not more than 50 mmol for Na+ or not more than 150 mmol for NH₄+.

## Revendications

1. Procédé pour le traitement intégré d'une biomasse provenant d'un processus de culture cellulaire, pour préparer un lysat cellulaire limpide, contenant un ADN plasmidique, **caractérisé en ce que** dans une première étape on procède à une filtration sur un filtre, dans une deuxième étape on soumet à un traitement thermique la biomasse contenue dans le gâteau de filtration, et dans une étape supplémentaire on filtre le lysat cellulaire, chaque étape étant effectuée en présence d'un auxiliaire de filtration inerte.

2. Procédé selon la revendication 1, **caractérisé en ce que** le traitement est mis en oeuvre à une température de 70 à 90 °C.

3. Procédé selon la revendication 1, **caractérisé en ce que** le traitement thermique est mis en oeuvre pendant au moins 30 secondes.

4. Procédé selon la revendication 1, **caractérisé en ce qu'**on utilise en tant qu'auxiliaire de filtration inerte des kieselguhrs ayant les propriétés suivantes :
- Teneur en dioxyde de silicium : ≥ 90 %
- Masse volumique à l'état humide : 0,2 à 0,4 g/cm³
- Perméabilité : 0,02 à 2 Darcy
- Aire BET : 2 à 20 m²/g
- Aire géométrique : 0,25 à 0,65 m²/g
- Granulométrie : 2,5 à 8,0 µm
- Rétention de granulométrie (99 %) : 0,1 à 2 µm.

5. Procédé selon les revendications 1 à 4, **caractérisé en ce qu'**on utilise le même auxiliaire de filtration dans les trois étapes.

6. Procédé selon les revendications 1 à 5, **caractérisé en ce que** le traitement est mis en oeuvre à un pH compris entre 7 et 10.

7. Procédé selon les revendications 1 à 6, **caractérisé en ce que** le traitement est mis en oeuvre en présence de cations exclusivement monovalents.

8. Procédé selon les revendications 1 à 7, **caractérisé en ce que** la concentration maximale des cations est d'au plus 20 mmoles pour K⁺, d'au plus 50 mmoles pour Na⁺ ou d'au plus 150 mmoles pour NH₄⁺.
